# EUROPÄISCHE PATENTANMELDUNG

(11) **EP 2 329 795 A1**
(43) Veröffentlichungstag der Anmeldung: **08.06.2011**
(21) Anmeldenummer: 10014640.6
(22) Anmeldetag: 16.11.2010
(51) Int. Cl.: A61F 2/12

(54) **Körperformendes Implantat**

(30) Priorität: 04.12.2009 DE 202009016559 U
(71) Anmelder: Peter Osypka Stiftung Stiftung des bürgerlichen Rechts, 79639 Grenzach-Wyhlen (DE)
(72) Erfinder: Osypka, Peter, Dr.-Ing., 79639 Grenzach-Wyhlen (DE)
(74) Vertreter: Maucher, Wolfgang

(57) **Zusammenfassung**

Ein zur Formung oder Ergänzung eines Körperteils darin einsetzbares Implantat (1) mit einer flexiblen, aber undurchlässigen Hülle (2) und mit einer elektrisch leitfähigen Füllung (3), verfügt über eine Innenelektrode (4) und eine Außenelektrode (5), die mit einer in einem Transponder (6) integrierten Widerstands- oder Impedanzmessvorrichtung verbunden sind. Tritt bei einer Perforation der Hülle (2) Füllung (3) aus, wird der elektrische Kontakt zwischen den Elektroden (4) und (5) geschlossen und es kann ein Abfall des Widerstands beziehungsweise der Impedanz von der Messvorrichtung detektiert werden. Mit einer externen Sender-Empfänger-Einheit (13) oder (14) kann das Implantat (1) im Verdachtsfall mittels der gemessenen Widerstands- oder Impedanzwerte kontrolliert und eine gefährliche Beschädigung des Implantats (1) festgestellt werden.(Fig.9)

## Beschreibung

Die Erfindung betrifft ein zur Formung oder Ergänzung eines Körperteils darin einsetzbares Implantat mit einer flexiblen, aber undurchlässigen Hülle und mit einer darin befindlichen Füllung.

Derartige Implantate sind seit langem in Form von Brust-, Waden-, Bizeps-, Bauchmuskel- oder Gesäßimplantaten bekannt. Trotz sorgfältiger Herstellung und Implantation kann es jedoch beispielsweise bei Unfällen dazu kommen, dass die Hülle des Implantats zumindest stellenweise durchlässig wird, einen Riss bekommt oder sonst eine Zerstörung aufweist, durch die Füllung austreten kann.

Mit dem Austreten der Füllung können sich medizinische Komplikationen einstellen. Neben ästhetischen Beeinträchtigungen wie Deformationen und Verhärtungen der bisherigen Füllung, die nicht selten weitere chirurgische Eingriffe notwendig machen, können ernste gesundheitlichen Schäden auftreten. Insbesondere kann das das Implantat umgebende Gewebe durch einen solchen Austritt der Füllung beeinträchtigt werden.

Eine Untersuchung bei entsprechenden Beschwerden, beispielsweise durch Ultraschall, Röntgen oder MRT, ist aufwendig und kostspielig.

Es besteht deshalb die Aufgabe, ein Implantat der eingangs definierten Art zu schaffen, bei welchem eine Beschädigung mit der Folge, dass Füllung austritt, möglichst einfach feststellbar ist.

Zur Lösung dieser Aufgabe ist das eingangs definierte Implantat dadurch gekennzeichnet, dass das Implantat in seinem Inneren wenigstens einen elektrischen Pol oder eine Elektrode und an seiner Außenseite wenigstens einen weiteren elektrischen Pol oder eine Elektrode aufweist, dass die beiden Pole oder Elektroden mit einer elektrischen Widerstands- oder einer Impedanzmessvorrichtung verbunden sind und dass die Füllung des Implantats elektrisch leitfähig ist.

Da die beiden Elektroden im Normalfall durch die Hülle des Implantats elektrisch voneinander getrennt sind, besteht eine sehr hohe Impedanz oder ein sehr hoher Widerstand. Tritt an der Hülle des Implantats ein Schaden auf, der zu einem Austritt eines Teils der elektrisch leitfähigen Füllung führt, ergibt sich zwischen den beiden Polen oder Elektroden über die im Implantat verbleibende Füllung, die durch den Schaden entstandene Öffnung der Hülle und den austretenden Füllungsteil sowie das Körpergewebe und/oder die Körperflüssigkeit ein wesentlich geringerer Widerstand, der dann als Signal für die Fehlerhaftigkeit oder Beschädigung des Implantats dienen kann. Die Anzeige des Widerstands- bzw. Impedanzabfalls könnte beispielsweise mittels eines akustischen Signals oder eines Vibrationsalarms erfolgen.

Die elektrisch leitfähige Füllung kann in an sich bekannter Weise eine Kochsalzlösung oder eine Gel-Füllung mit sie leitfähig machenden Zusätzen sein. Solche Zusätze können zum Beispiel Carbon- oder Metallpulver sein.

Für eine möglichste einfache Kontrolle des Implantats und der dafür notwendigen Übermittlung der Änderung der Widerstands-oder Impedanzwerte ist es besonders günstig, wenn die beiden Pole oder Elektroden mit einem die Messvorrichtung enthaltenen Transponder verbunden sind, der zur Übertragung der Messwerte auf einen externen Empfänger ausgebildet ist.

Somit genügt es für die Überprüfung des Implantats beispielsweise bei dem Verdacht, dass eine Beschädigung seiner Hülle vorliegt, einen externen Empfänger in die Reichweite des Transponders zu bringen und die Widerstands- oder Impedanzwerte auszulesen.

Eine Möglichkeit zur Gestaltung des Implantats könnte vorsehen, dass der Transponder eine Widerstandsmessbrücke enthält. Damit kann der für den Transponder und die Messvorrichtung notwendige Bauraum im oder am Implantat möglichst klein gehalten werden.

Um die an die natürliche Anatomie angepasste Form des eingesetzten Implantats trotz der angebrachten Pole oder Elektroden, des Transponders und der Messvorrichtung zu bewahren, ist es besonders günstig, wenn der im Inneren des Implantats befindliche elektrische Pol oder die Elektrode an der in Gebrauchsstellung rückwärtigen Wandung oder Rückseite des Implantats innenseitig angeordnet oder befestigt ist. Unter Rückseite wird dabei die in Gebrauchslage von der Körperaußenseite abgewandte Seite, bei einem Brustimplantat die den Rippen zugewandte Seite, verstanden.

In gleicher Weise kann es zweckmäßig sein, wenn der außen befindliche Pol oder die Elektrode an der in Gebrauchsstellung rückwärtigen Wandung oder Rückseite des Implantats an dessen Außenseite angeordnet oder befestigt ist. Einerseits wird dadurch die Anatomie nicht sichtbar beeinflusst und andererseits wird die natürliche Nach-giebigkeit des Implantats an der es enthaltenden Stelle des Körpers nicht oder kaum beeinträchtigt.

Zweckmäßig ist es, wenn die beiden Pole oder Elektroden durch die Wandung des Implantats voneinander elektrisch getrennt und/oder zusätzlich gegeneinander isoliert sind. Entsprechend sicher kann die Detektion eines Austretens von elektrisch leitfähiger Füllung durch eine Verminderung des messbaren Widerstandes oder der messbaren Impedanz erfolgen.

Eine weitere Ausgestaltung der Erfindung kann vorsehen, dass die beiden Pole oder Elektroden an den sich gegenüberliegenden Außenseiten des Transponders befestigt sind und mit diesem eine Einheit bilden.

Dadurch lassen sich die Elektroden, der Transponder und die Messvorrichtung in einem vorgelagerten Arbeitsschritt zu einer Einheit verbinden, die anschließend in bestimmungsgemäßer Art und Weise in oder an dem Implantat befestigt wird.

Besonders günstig ist es, wenn die die außenliegende Elektrode oder den außenliegenden Pol aufnehmenden Stelle oder Wandung eine Vertiefung für den Pol oder die Elektrode aufweist und der Pol oder die Elektrode in Gebrauchsstellung gegenüber der Oberfläche der Außenseite wenigstens teilweise oder derart eingesenkt ist, dass die Außenseite des Pols oder der Elektrode mit der Außenseite der ihn aufnehmenden Wandung bündig ist.

Damit ragt die Außenelektrode nur wenig oder gar nicht aus der Oberfläche des Implantats heraus und unerwünschte Veränderungen hinsichtlich seiner bestimmungsgemäßen Form durch die Außenelektrode können vermieden werden.

Eine für die Anwendung des Implantats vorteilhafte Ausgestaltung der Erfindung kann darin bestehen, dass an der Rückwand des Implantats eine Öffnung für das Einbringen der Implantatsfüllung und ein Verschluss dafür vorgesehen ist und dass der außenliegende Pol oder die Elektrode, der Transponder und der innenliegende Pol oder die Elektrode an oder in dem Verschluss angeordnet sind.

Mit dieser kompakten Bauweise und der Integration der beiden Elektroden und des Transponders in den Verschluss können ungewünschte Einflüsse auf die Form des Implantats vermieden und die Herstellung vereinfacht werden.

Um eine schnelle und möglichst sichere Detektion des Austretens von Füllung aus dem Implantat feststellen zu können, können mit dem außenliegenden Pol oder der Elektrode von diesem oder dieser ausgehende Leitungen oder elektrisch leitende Arme verbunden sein, die zumindest an der in Gebrauchsstellung rückwärtigen Außenseite der Rückwand des Implantats angeordnet sind oder bis über die Vorderseite des Implantats reichen.

Dementsprechend ergeben sich kürzere Wege, bis ausgetretenes Füllungsmaterial in Kontakt mit Teilen der Außenelektrode tritt und somit zu einem Rückgang des Widerstandes oder der Impedanz führt. Je näher sich die außenliegende Elektrode oder Teile von ihr an der Austrittsstelle befinden, desto schneller kann das Austreten von Füllung und die Beschädigung durch den Rückgang des gemessenen Widerstandes oder der Impedanz festgestellt werden.

Die Leitungen oder elektrisch leitenden Arme können auch in Form von elektrisch leitenden Metallwendeln ausgeführt sein.

Eine weitere Ausgestaltung der Erfindung kann vorsehen, dass die zusätzlichen elektrisch leitenden Arme als nachgiebig verformbare, elektrisch leitfähige Bänder ausgebildet sind.

Ein derart gestaltetes Implantat ermöglicht es, sämtliche für die Implantatüberwachung notwendigen Elemente so zu installieren, dass sich die Außenform des Implantats nicht oder nur gering von einem solchen Implantat ohne diese Vorrichtung unterscheidet. Solche Bänder beeinflussen folglich nicht die Form des Implantats, können aber große Bereiche seiner Oberfläche abdecken und somit die Distanzen zwischen der etwaigen Austritts- und Versagensstelle und sich selbst reduzieren und damit die Dauer zwischen Austritt von Füllung und Detektion durch die Überwachungsvorrichtung verringern.

Nachstehend sind Ausführungsbeispiele der Erfindung anhand der Zeichnungen näher beschrieben. Es zeigt in zum Teil schematisierter Darstellung:
- Fig. 1: eine Vorderansicht eines erfindungsgemäßen, hier transparent dargestellten Implantats mit einer undurchlässigen Hülle, einer elektrisch leitfähigen Füllung und den teilweise sichtbaren Polen oder Elektroden,
- Fig. 2: eine Seitenansicht des Implantats gemäß Fig. 1,
- Fig. 3: eine der Fig. 2 entsprechende Darstellung ergänzt um die schematische Abbildung eines mit-implantierten Transponders, der eine Widerstands- oder Impedanzmessvorrichtung enthält,
- Fig. 4: eine vergrößerte Seitenansicht der Anordnung der Pole oder Elektroden an der rückwärtigen Wandung oder Rückseite des Implantats,
- Fig. 5: eine der Fig.1 entsprechende Darstellung einer abgewandelten Ausführungsform, bei welcher elektrisch leitfähige Arme oder Bänder in vertikaler und horizontaler Richtung über die Rückwand des Implantats verlaufen,
- Fig. 6: eine der Fig. 5 entsprechende Darstellung einer abgewandelten Ausführungsform, bei der zusätzlich ein elektrisch leitfähiges Band an der rückseitigen Fläche des Implantats randnah umläuft,
- Fig. 7: .eine der Fig. 1 entsprechende Darstellung einer abgewandelten Ausführungsform, bei der die elektrisch leitfähigen Bänder bis über die Vorderseite des Implantats geführt sind,
- Fig. 8: eine der Fig. 7 entsprechende Darstellung in Seitenansicht,
- Fig. 9: ein erfindungsgemäßes Implantat in Gebrauchsstellung und eine Sender-Empfänger-Einheit zum Zusammenwirken mit einem passiven Transponder sowie
- Fig. 10: eine der Fig. 9 entsprechende Darstellung in der Ausführung mit einem aktiven Transponder.

Ein im Ganzen mit 1 bezeichnetes in den Ausführungsbeispielen als Brustimplantat ausgeführtes Implantat weist eine flexible, aber undurchlässige Hülle 2 und eine darin befindliche, elektrisch leitfähige Füllung 3 auf. In seinem Inneren sind wenigstens ein elektrischer Pol oder eine Elektrode 4, im Weiteren "Innenelektrode" genannt, und an seiner Außenseite wenigstens ein weiterer elektrischer Pol oder eine Elektrode 5, im Weiteren als "Außenelektrode" bezeichnet, angeordnet. Die beiden Elektroden 4 und 5 sind mit einer nicht gesondert dargestellten elektrischen Widerstands- oder einer Impedanzmessvorrichtung, im Folgenden "Messvorrichtung" genannt, verbunden.

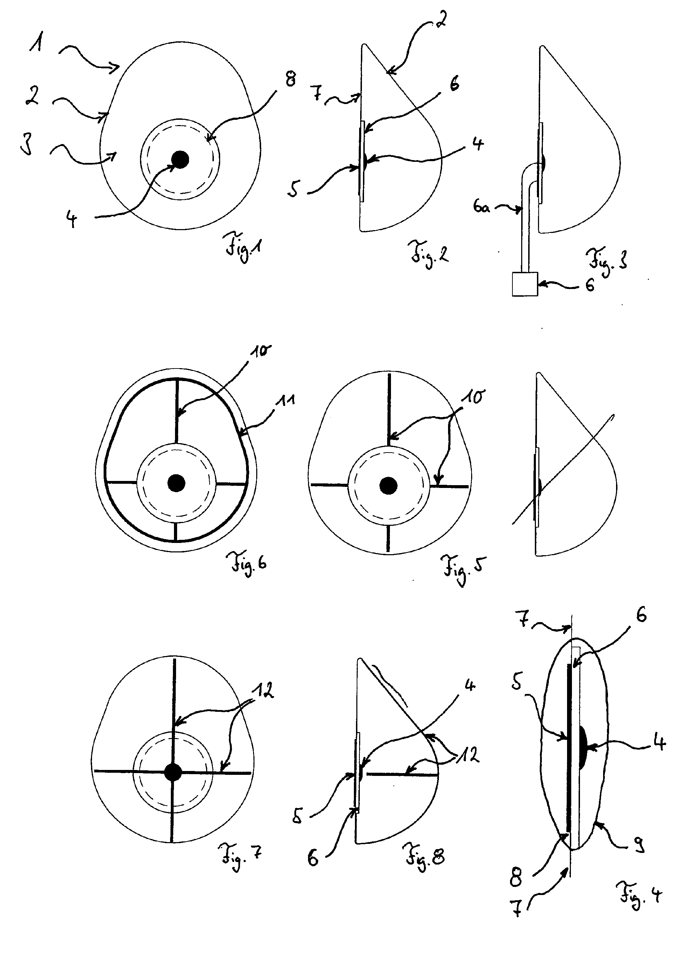

Die Messvorrichtung kann dabei in einen Transponder 6 integriert sein, der sich gemäß Figur 3 über Leitungen 6a außerhalb des Implantats 1 oder gemäß den Figuren 2, 4 und 8 bis 10 unmittelbar an dem Implantat 1 befinden kann.

In den Figuren 2, 4 und 8 bis 10 sind Ausführungsbeispiele dargestellt, bei denen die Lage der beiden Elektroden 4 und 5 im Verhältnis zum Transponder 6 ersichtlich wird.

Die beiden Elektroden 4 und 5 sind an den sich gegenüberliegenden Außenseiten des Transponders 6 befestigt und bilden mit diesem eine Einheit. Der Transponder 6 befindet sich somit zwischen den beiden Elektroden 4 und 5.

Diese Einheit wird so an beziehungsweise in der Rückwand 7 des Implantats 1 befestigt, dass die Innenelektrode 4 an der dem Implantat 1 zugewandten Innenseite und die Außenelektrode 5 an der dem Implantat 1 abgewandten Außenseite des Transponders 6 sitzt.

Durch die Hülle 2 des Implantats 1 und den Transponder 6 werden die Elektroden 4 und 5 elektrisch voneinander getrennt.

Wie die Figuren 1 bis 8 zeigen, kann das Implantat 1 eine Öffnung 8 zum Einbringen der Implantatfüllung 3 auf seiner Rückseite 7 haben. Diese Öffnung 8 kann durch einen in Figur 4 vergrößert dargestellten Verschluss 9 abgedichtet werden.

Der Verschluss 9 enthält den Transponder 6, auf dem implantat-innenseitig die Innenelektrode 4 und implantat-außenseitig die Außenelektrode 5 angebracht ist.

Nach Einbringen der Implantatfüllung 3 wird der Verschluss 9 eingesetzt und befestigt, und dichtet die Öffnung 8 des Implantats 1 ab, beispielsweise durch Verkleben mit der Rückseite 7 des Implantats 1.

Figur 5 zeigt zur Vergrößerung der Kontaktfläche der Außenelektrode 5 zusätzliche elektrisch leitende Arme oder Bänder 10, die in horizontaler und vertikaler Richtung auf der Rückwand 7 des Implantats verlaufen.

Eine weitere Ausgestaltung der Außenelektrode zeigt Figur 6. An die elektrisch leitenden Arme 10 schließt sich ein, die Fläche der Rückwand 7 randnah umlaufendes, elektrisch leitfähiges Band 11 an.

Die Kontaktfläche der Außenelektrode 5 lässt sich, wie in den Figuren 7 und 8 dargestellt, weiter vergrößern, indem die elektrisch leitenden Arme 12 bis auf die Vorderseite des Implantats geführt werden und auf dieser in horizontaler und vertikaler Richtung verlaufen.

Tritt bei einer Perforation der Hülle 2 des Implantats 1 elektrisch leitfähige Füllung 3 nach außen, schließt sich der elektrische Kontakt zwischen Innenelektrode 4 und der Außenelektrode 5 über die Füllung und/oder über Körpergewebe oder -flüssigkeit. Dabei verzeichnet die Messvorrichtung einen Abfall des Widerstandes beziehungsweise der Impedanz.

Bei den Ausführungsbeispielen gemäß den Figuren 5 bis 8 ermöglichen die zusätzlichen elektrisch leitfähigen Arme 10, 11 und 12 eine frühere Kontaktierung der beiden Elektroden und damit eine frühere Detektion des Austritts der Implantatfüllung 3. Über den Transponder 6 kann der gemessene Impedanz- oder Widerstandswert an einen noch zu erläuternden externen Empfänger gesendet werden.

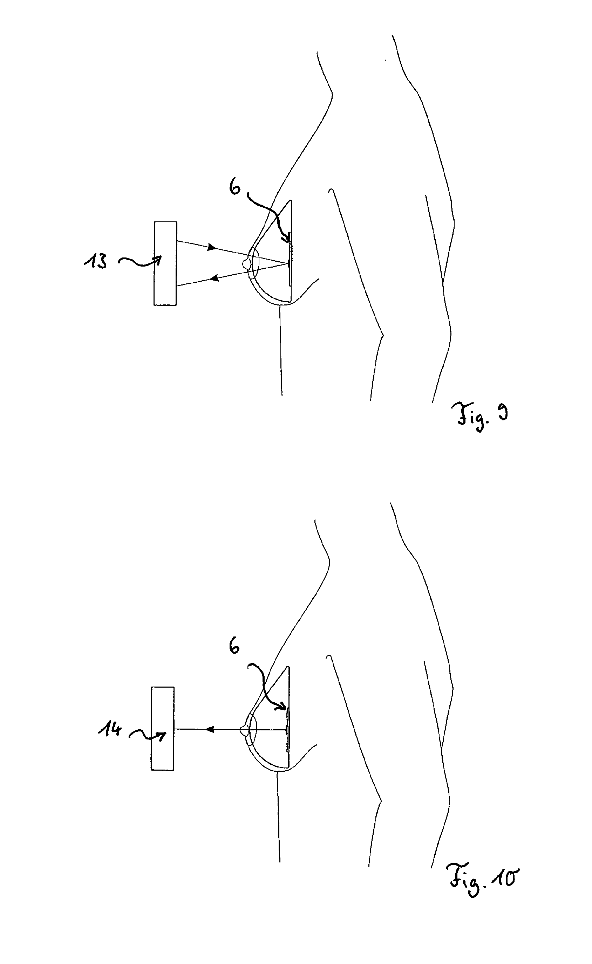

Figur 9 zeigt schematisiert einen Empfänger 13, der mit einem Sender eine Einheit bildet, im Zusammenwirken mit dem Transponder 6. Der Transponder 6 ist hier ein passiver Transponder, verfügt also über keine eigene Stromversorgung, sondern wird über das elektromagnetische Feld der Sender-Empfänger-Einheit gespeist. Sobald er angeregt wird, kann er die gemessenen Impedanz- oder Widerstandswerte an den externen Sender-Empfänger 13 übertragen.

Figur 10 zeigt eine abgewandelte Ausführung bei der ein aktiver Transponder 6 über eine eigene, hier nicht weiter dargestellte Energieversorgung, zum Beispiel eine Batterie, verfügt. Der Transponder 6 kann folglich auch ohne Anregung beziehungsweise Speisung aus dem elektromagnetischen Feld einer Sender-Empfänger-Einheit Daten und Messwerte an den externen Empfänger 14 übertragen und so bei Austritt von Füllung 3 die Beschädigung der Hülle 2 des Implantats 1 anzeigen.

## Patentansprüche

1. Zur Formung oder Ergänzung eines Körperteils darin einsetzbares Implantat (1) mit einer flexiblen, aber undurchlässigen Hülle (2) und mit einer darin befindlichen Füllung (3), **dadurch gekennzeichnet, dass** das Implantat (1) in seinem Inneren wenigstens einen elektrischen Pol oder eine Elektrode (4) und an seiner Außenseite wenigstens einen weiteren elektrischen Pol oder eine Elektrode (5) aufweist, dass die beiden Pole oder Elektroden mit einer elektrischen Widerstands- oder einer Impedanzmessvorrichtung (6) verbunden sind und dass die Füllung (3) des Implantats (1) elektrisch leitfähig ist.

2. Implantat nach Anspruch 1, **dadurch gekennzeichnet, dass** die beiden Pole oder Elektroden (4, 5) mit einem die Messvorrichtung enthaltenen Transponder (6) verbunden sind, der zur Übertragung der Messwerte auf einen externen Empfänger (13, 14) ausgebildet ist.

3. Implantat nach einem der Ansprüche 1 oder 2, **dadurch gekennzeichnet, dass** der Transponder (6) eine Widerstandsmessbrücke enthält.

4. Implantat (1) nach Anspruch 1 bis 3, **dadurch gekennzeichnet, dass** der im Inneren des Implantats (1) befindliche elektrische Pol oder die Elektrode (4) an der in Gebrauchsstellung rückwärtigen Wandung oder Rückseite (7) des Implantats (1) innenseitig angeordnet oder befestigt ist.

5. Implantat (1) nach Anspruch 1 bis 4, **dadurch gekennzeichnet, dass** der außen befindliche Pol oder die Elektrode (5) an der in Gebrauchsstellung rückwärtigen Wandung oder Rückseite (7) des Implantats (1) an dessen Außenseite angeordnet oder befestigt ist.

6. Implantat (1) nach einem der Ansprüche 1 bis 5, **dadurch gekennzeichnet, dass** die beiden Pole oder Elektroden (4, 5) durch die Hülle (2) des Implantats (1) voneinander elektrisch getrennt und/oder zusätzlich gegeneinander isoliert sind.

7. Implantat (1) nach einem der Ansprüche 1 bis 6, **dadurch gekennzeichnet, dass** die beiden Pole oder Elektroden (4, 5) an den sich gegenüberliegenden Außenseiten des Transponders (6) befestigt sind und mit diesem eine Einheit bilden.

8. Implantat (1) nach einem der Ansprüche 1 bis 7, **dadurch gekennzeichnet, dass** es an der die außenliegende Elektrode oder den außenliegenden Pol (5) aufnehmenden Stelle oder Wandung eine Vertiefung für den Pol oder die Elektrode aufweist und der Pol oder die Elektrode (5) in Gebrauchsstellung gegenüber der Oberfläche der Außenseite wenigstens teilweise oder derart eingesenkt ist, dass die Außenseite des Pols oder der Elektrode mit der Außenseite der ihn aufnehmenden Wandung bündig ist.

9. Implantat (1) nach einem der Ansprüche 1 bis 8, **dadurch gekennzeichnet, dass** an der Rückwand (7) des Implantats (1) eine Öffnung (8) für das Einbringen der Implantatsfüllung und ein Verschluss (9) dafür vorgesehen ist und dass der außenliegende Pol oder die Elektrode (5), der Transponder (6) und der innenliegende Pol oder die Elektrode (4) an oder in dem Verschluss (9) angeordnet sind.

10. Implantat (1) nach einem der Ansprüche 1 bis 9, **dadurch gekennzeichnet, dass** mit dem außenliegenden Pol oder der Elektrode (5) von diesem oder dieser ausgehende Leitungen oder elektrisch leitende Arme (10,11,12) verbunden sind, die zumindest an der in Gebrauchsstellung rückwärtigen Außenseite der Rückwand (7) des Implantats (1) angeordnet sind oder bis über die Vorderseite des Implantats (1) reichen.

11. Implantat (1) nach einem der Ansprüche 1 bis 10, **dadurch gekennzeichnet, dass** die zusätzlichen elektrisch leitenden Arme als nachgiebig verformbare, elektrisch leitfähige Bänder ausgebildet sind.
